# EUROPEAN PATENT SPECIFICATION

(11) **EP 0 475 623 B1**
(45) Date of publication and mention of the grant of the patent: **17.04.1996**
(21) Application number: 91307791.3
(22) Date of filing: 23.08.1991
(51) Int. Cl.: A61K 48/00, A61K 38/17, C12N 15/11, C07H 21/00, C07H 13/00

(54) **Genetic mechanisms of tumor supression**
Genetische Mechanismen der Tumorunterdrückung
Mécanismes génétiques de suppression de tumeurs

(30) Priority: 24.08.1990 US 573405
(43) Date of publication of application: 18.03.1992
(60) Divisional application: 95114723.0
(73) Proprietor: THE REGENTS OF THE UNIVERSITY OF CALIFORNIA, Oakland, California 94612-3550 (US)
(72) Inventor: Lee, Wen-Hwa, San Diego, CA 92130 (US); Chen, Phang-Lang, San Diego, CA 92122 (US)
(74) Representative: Spall, Christopher John

(56) References cited:
- SCIENCE, vol. 247, 09 February 1990, Washington, DC (US); R. BOOKSTEIN et al., pp. 712-715
- CHEMICAL ABSTRACTS, vol. 107, no. 25, 21 December 1987, Columbus, OH (US); T. SOUSSI et al., p. 208, no. 230321n
- CHEMICAL ABSTRACTS, vol. 106, no. 9, 02 March 1987, Columbus, OH (US); N. HARRIS et al., p. 143, no. 62030a
- MOLECULAR & CELLULAR BIOLOGY, vol. 6, no. 5, May 1986, Washington, DC (US); P. LAMB et al., pp. 1379-1385
- CHEMICAL ABSTRACTS, vol. 103, no. 19, 11 November 1985, Columbus, OH (US); R. ZAKUT-HOURI et al., pp. 204-205
- CHEMICAL ABSTRACTS, vol. 103, no. 15, 14 October 1985 Columbus, OH (US); D. WOLF et al., p. 190, no. 117348f
- CHEMICAL ABSTRACTS, vol. 103, no. 11, 16 September 1985 Columbus, OH (US); E. HARLOW et al., p. 156, no. 82693k
- CHEMICAL ABSTRACTS, vol. 102, no. 13, 01 April 1985, Columbus, OH (US); G. MATLASHEWSKI et al., p. 175, no. 107319n

## Description

This invention relates in general to cell therapy and to methods for treating cells to suppress tumorigenesis.

This invention was made with Government support under Grant No. EY05758 with the National Institute of Health, and the University of California. The Government has certain rights in this invention.

### Background Art

Much of the focus of cancer research has been on the diagnosis and treatment of the condition. In recent years, because of advances in knowledge of biochemical processes at the cellular and subcellular levels, attention has been directed to methods, not only for diagnosing and treating cancer, but also for discovering a predisposition for cancer in the organism.

In these studies, "cancer suppression" was originally defined by a loss of tumorigenicity observed in fusion cells made with tumor cells and normal fibroblasts, lymphocytes or keratinocytes. The effect was presumed to be mediated by dominant suppressive factors in normal cells. Evidence indicated that these factors were in part genetic since a correlation existed between suppression of tumorogenicity and the presence of certain chromosomes in fused cells.

Another meaning for cancer suppressing genes arose in connection with genetic studies on certain childhood neoplasms and adult tumor syndromes. Genes contributing to the formation of these tumors appear to be oncogenic by loss of function, rather than activation, as with the classical oncogenes. Retinobastoma, a childhood eye cancer, has provided the prototypic example. Refined cytogenic analysis and study of restriction fragment length polymorphisms (RFLPs) have suggested that retinobastoma may result from a loss of a gene locus located in chromosome band 13q14. Significant advances have been made in the utilization of RB cDNA and RB protein not only in diagnosis and methods of treatment of RB-related tumors, but also in the elucidation of the cancer suppressor functions of other genes. Nevertheless, a significant need exists for appropriate methods for the therapeutic treatment of osteosarcoma, lung carcinoma, lymphomas and leukemias, which are not aminable to treatment by RB modalities.

In view of the above, it would be highly desirable to have a method for specific therapeutic treatment, independent of RB modalities, for osteosarcomas, lung carcinomas, lymphomas and leukemias. Further, it would be highly desirable to have such methods which could be used in conjunction with RB cDNA and protein product for the treatment of various tumors. Of course, it would be highly desirable to have such a therapeutic product which could be made in a purified state and which would be readily and effectively deliverable to a defective cell in a safe manner.

### Disclosure of Invention

It is a primary object of this invention to provide generally safe and specific therapeutic methods and products useful for controlling cancer suppression. It is a further object of this invention to provide products and methods for controlling cancer suppression which are specific for suppression and eradication of cancer tumors and which utilize biotechnological methods and products.

It is a still further object of the present invention to provide a pharmaceutical composition for therapeutically treating cancers wherein the composition is functional at the cellular and intracellular levels.

It is yet still another object of the present invention to provide a pharmaceutical composition for treating conditions caused by defective, mutant or absent cancer suppressor genes wherein the active ingredient of the composition is a natural or synthetically produced product.

The present invention provides a method for utilizing p53 cDNA, and p53 gene products, for the suppression of the neoplastic phenotype.

### Brief Description of Drawings

The above mentioned and other objects and features of this invention and the manner of attaining them will become apparent, and the invention itself will be best understood by reference to the following description of the embodiment of the invention in conjunction with the accompanying drawings, wherein:
FIG. 1A is a diagrammatic representation of three human p53cDNAs;
FIG. 1B is a diagrammatic representation of the genomic organization of three p53 retroviruses;
FIG. 2A is a chromatogram depicting expression of human p53 proteins in virus-producing cell lines;
FIG. 2B is a chromatogram depicting half-life determination of human p53 in virus-producing lines by pulse-chase labeling experiments;
FIG. 3 depicts the expression of human p53 proteins in virus-infected Saos-2 cells;
FIG. 4 is a chromatogram of p53B/T complex formation in Saos-2 cells;
FIG. 5 depicts morphology in a culture of parental Saos-2 cells;
FIG. 6A is a schematic representation of the doubling times of parental Saos-2 cells and virus-infected clones;
FIG. 6B is a schematic representation of the saturation density of parental Saos-2 and EN clones;
FIG. 7A is a Southern blot depicting the presence of a single integrated copy of Vp53E-Neo in p53EN-1 cells; and
FIG. 7B is a Southern blot depicting single, independently integrated copies of Vp53B-Hygro in p53EN-BH clones.

### Detailed Description of Figures

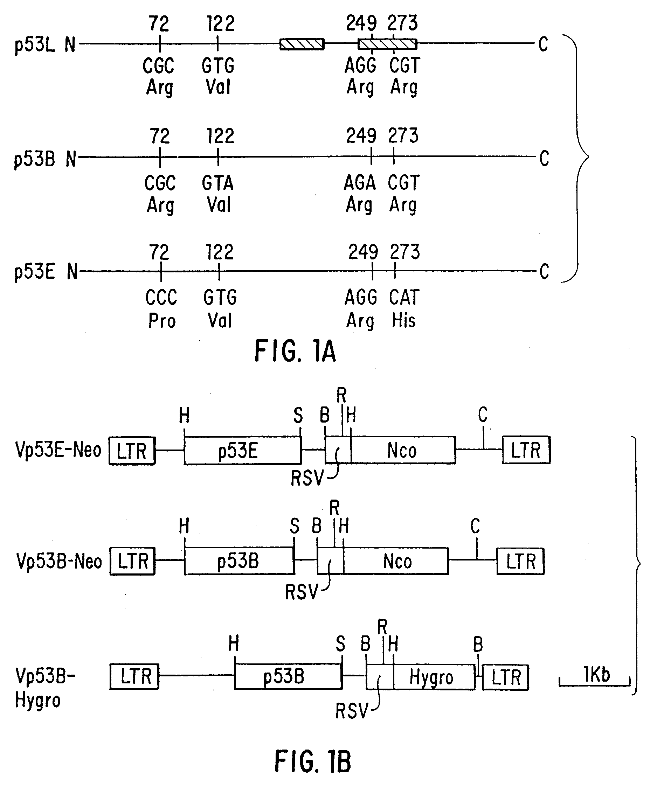

In FIG. 1A, three human p53 cDNAs are diagrammed. The sequence reported by Lam and Crawford, Mol. Cell. Biol. 6, 1379-1385 (1986), here labelled as p53L, was derived by sequencing clones from human fetal liver cDNA and genomic libraries, and is considered to be wild-type. p53B is a cDNA clone derived from fetal brain RNA by the RT-PCR method which resulted in cloning of wild type p53 (p53B) cDNA as follows: about 15 µg of fetal brain RNA were mixed with 1.5 µg of oligo (dT) primer and 60 units of avian myeloblastosis virus reverse transcriptase in cDNA buffer (50 mM Tris-HCl, pH 8.0, 80 mM KCl, 5 mM MgCl2, 1mM each dATP, dGTP, dTTP, and dCTP). The reaction mixture was incubated for 90 min at 42°C. After reaction, RNA was degraded with 0.5N NaOH, and single-stranded cDNA was precipitated with ethanol. PCR amplification was carried out with one-tenth of the cDNA, 100 ng of each oligonucleotide primer (5'-TGCAAGCTTTCCACGACGGTGACACGCT-3' and 5'-AGTGCAGGCCAACTTGTTCAGTGGA-3'), and 5U of Taq polymerase in PCR buffer (50 mM KCl, 10 mM Tris-HCl, pH 8.3, 1.5 mM MgCl2, and 0.001% gelatin) for 40 cycles in a programmable heat block (Ericomp, San Diego, CA). Each cycle included denaturation at 93°C for 1 minute, reannealing at 62°C for 60 seconds, and primer extention at 72°C for 3 minutes. PCR products were extracted with phenol and precipitated with ethanol. The precipitate was dissolved in H₂0 and digested with restriction enzymes (Hind III and Sma 1). The p53cDNA fragment was subcloned into virus vector to form Vp53B-Neo. Subcloned p53B was sequenced by using the dideoxy chain termination method (F. Sanger, S. Nicklen, A.R. Coulsen, Proc. Natl. Acad. Sci. U.S.A. 74, 5463 (1977)).

The deducted amino acid sequences of p53B and p53L were identical despite two silent nucleotide substitutions as indicated. p53E is a cDNA clone provided by E. Harlow, that has amino acid substitutions at positions 72 and 273 relative to p53L or p53B. The Arg/Pro⁷² replacement represents a common amino acid polymorphism, without known functional significance. On the other hand, the substitution of His for Arg at position 273 is found exclusively in tumor cells and is considered to be a mutation. Like many other p53 mutations, Arg ²⁷³--> His lies within one of two regions required for binding to SV40 T antigen (hatched boxes).

In FIG. 1B, the genomic organization of three p53 retroviruses are diagrammed. Vp53E-Neo was constructed by inserting a 1.5 kb Hind III-Sma I DNA fragment containing p53E into the plasmid pLRbRNL, replacing RB cDNA. A 1.35 kb p53B DNA obtained by RT-PCR was directly inserted into the pLRbRNL vector to form Vp53B-Neo. The insert in one clone was entirely sequenced, as diagrammed in FIG. 1A. Vp53B-Hygro was constructed by insertion of a Hind III DNA fragment containing p53B and the RSV promoter into plasmid 477 (a MuLV-Hygro vector kindly provided by W. Hammerschmidt and B. Sugden). These constructs were then used to produce the corresponding viral stocks using conventional techniques. Some major restriction sites important for construction are indicated. H = Hind III, R = EcoR I, S = Sma I, B - Bam HI, C = Cla I.

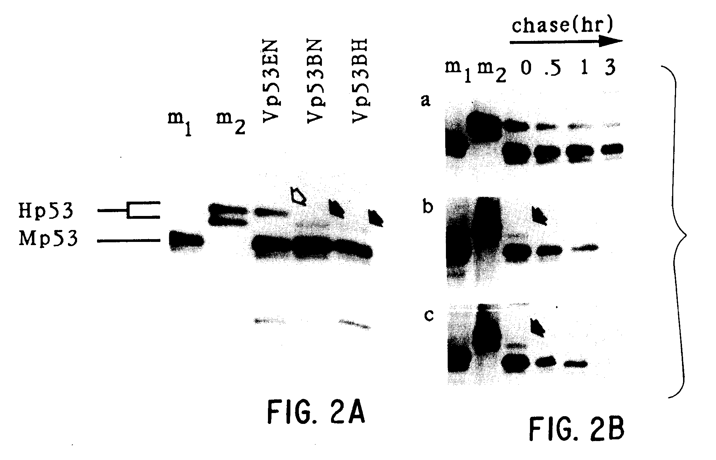

FIG. 2A is a chromatogram depicting Murine PA 12 cells (Lane ml), human WERI-Rb27 retinoblastoma cells (Lane m2), and Vp53En-, Vp53BN-, or Vp53BH-producing PA 12 cells which were metabolically labelled with ³⁵-methionine. Cell lysates were immunoprecipitated with anti-p53 antibody PAb421 utilizing conventional methods. Immunoprecipitates were separated by 8.5% SDS-polyacrylamide gel electrophoresis (SDS-PAGE), and autoradiographed. marker lanes m1 and m2 show endogenous murine p53 (Mp53) and both polymorphic forms of human p53 (Hp53). Human p53B (filled arrow) and p53E (open arrow) proteins in mouse cells are indicated.

FIG. 2B depicts half-life determination of human p53 in virus-producing lines by pulse-chase labeling experiments. PA12 cells expressing p53E (panel a) or p53B (panels b & c, representing two independent clones) were labelled with 0.25 mCi/ml ³⁵S-methionine for 60 minutes, and chased with a 1000-fold molar excess of unlabeled methionine. At the indicated times, cells were harvested for immunoprecipitation of p53 protein with PAb421 as described above. The half-life of p53B was 20-30 minutes whereas that of p53E was 4-5 hours. Marker lanes m1 and m2, and filled and open arrows, were as in FIG. 2A.

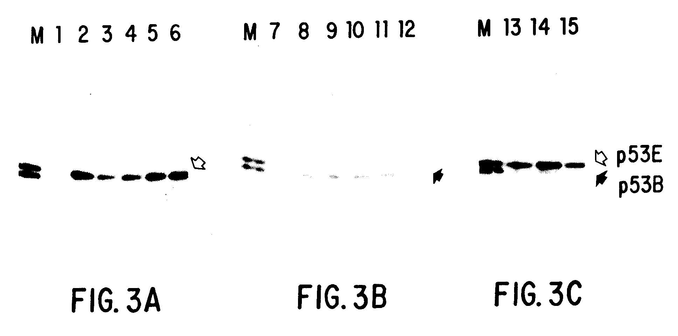

FIG. 3 is a chromatogram depicting expression of human p53 proteins in virus-infected Saos-2 cells. Saos-2 cells (lanes 1 and 7) were infected with Vp53E-Neo, Vp53B-Neo, or Vp53B-Hygro to generate p53EN (lanes 2-6), p53BN (lanes 8-10), or p53BH (lanes 11 and 12) clones, respectively. Saos-2 cells were also doubly infected with Vp53E-Neo and Vp53B-Hygro to generate p53EN-BH clones (lanes 13-15). Randomly-selected clones, and WERI-Rb27 cells (lanes M), were labeled with ³⁵S-methionine and immunoprecipitated with PAb421 as described with regard to FIG. 2A. p53B (filled arrows) and p53E (open arrows) are indicated.

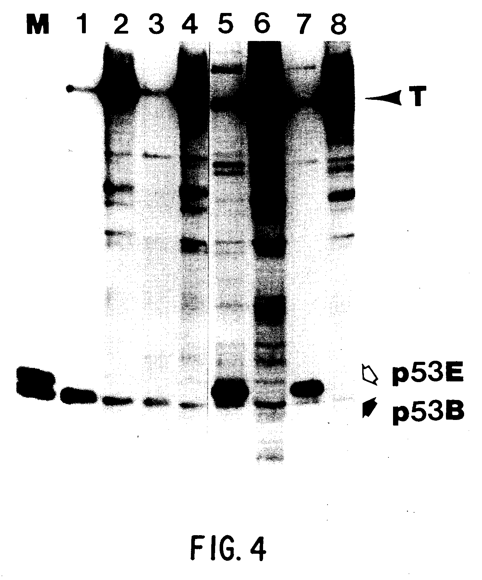

FIG. 4 depicts p53B/T complex formation in Saos-2 cells. Clones p53BN-1 (lanes 1 and 2), p53BH-1 (lanes 3 and 4), p53EN-1-BH-1 (lanes 5 and 6), and p53EN-1-BH-2 (lanes 7 and 8) were transfected with plasmid pRSV40T by conventional methods, and 60 hours later, were metabolically labelled with ³⁵S-methionine. Cell lysates were immunoprecipitated with PAb421 (lanes M, 1, 3, 5 and 7) or with PAb419, a monoclonal antibody against SV40T antigen (lanes 2, 4, 6 and 8). PAb419 coprecipitated only p53B in cells expressing both p53B and p53E.

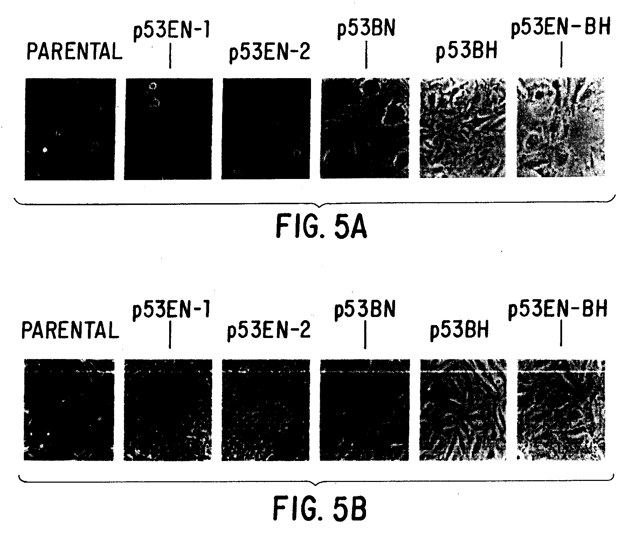

FIG. 5 is a photograph depicting morphology in culture of parental Saos-2 cells, and representative virus-infected clones at magnification 100x. In row A, exponentially growing cells were shown while in row B, cells at confluency are shown.

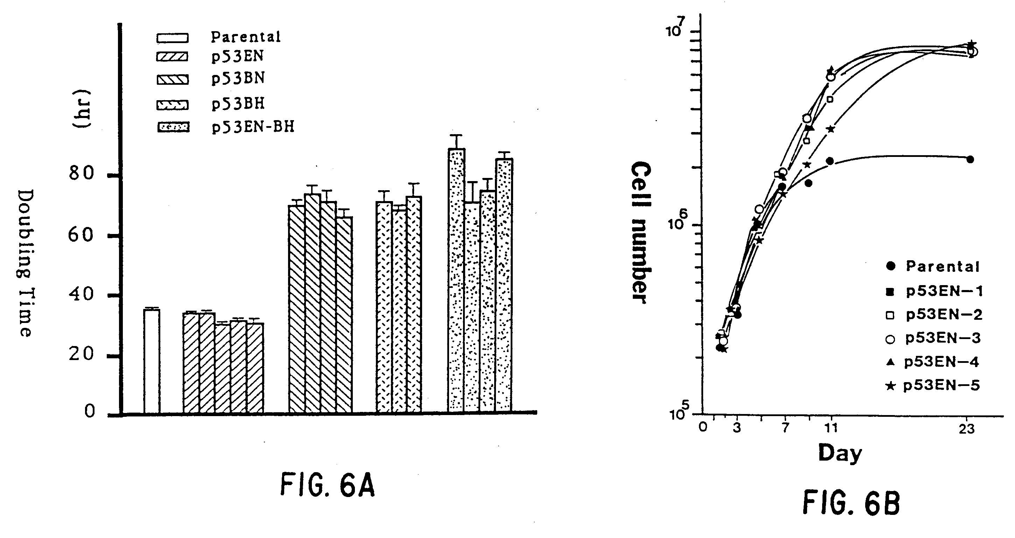

FIGS. 6A and 6B are schematic representations of growth effects of p53 expression in Saos-2 cells. In FIG. 6a, the doubling times of parental Saos-2 cells and virus-infected clones in an exponential growth stage are shown. Equal numbers of each cell type were seeded into 60 mm culture dishes; cells of two dishes were trypsinized and counted at daily intervals for 4 days. Doubling times were derived from lines fitted to log cell numbers. FIG. 6B shows the saturation density of parential Saos-2 and En clones. Equal numbers (1 x 10⁵) of cells were seeded into 60mm culture dishes; cells of two dishes were trypsinized and counted at the times indicated. Plotted points were mean cell numbers from duplicate dishes. Saturation density of p53E- expressing cells was 4- to 5- fold greater than parental cells.

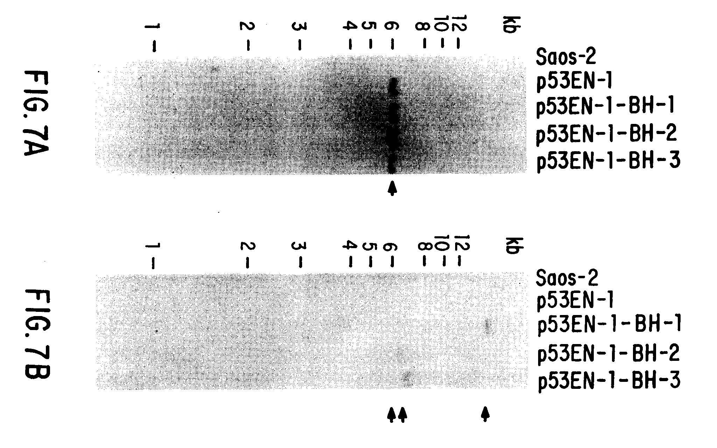

FIG. 7 is a Southern blot of p53EN-BH cells which harbored one copy of Vp53E-Neo and one copy of Vp53B-Hygro. Genomic DNA (10µg) extracted from parental Saos-2 cells and the indicated clones was digested with EcoR I, and separated in 0.7% agarose gels. Southern transfer was performed, and nylon membranes were hybridized with ³²p-labelled neo (panel A) or hygro (panel B) DNA probes, utilizing standard methods. A single junctional fragment is seen in each clone with each probe, indicating single integrated copies of each virus.

### Best Mode for Carrying Out the Invention

Tumor suppressor genes are defined as genes for which loss-of-function mutations are oncogenic. It is recognized that wild-type alleles of such genes may function to prevent or suppress oncogenesis. The retinoblastoma gene (RB) is the prototype of this class. Both alleles of this gene are invariably mutated in retinoblastoma cells, and RB mutations are also found in a subset of other human neoplasms including osteosarcoma, soft-tissue sarcomas, and carcinomas of breast, lung, bladder and prostate. Introduction of a wild-type copy of RB into retinoblastoma cells suppressed their tumorigenic properties in nude mice, thereby providing direct evidence for tumor suppression by a single gene. The wild-type RB gene was also introduced into human prostate carcinoma cells containing an endogenous, nutated RB protein (Science 247, 712-715 (1990)). Expression of the exogenous gene again suppressed the tumorigenicity of these cells, implying that wild-type RB protein was phenotypically dominant to the mutated form. These results support a general model for the properties of tumor suppressor genes that has emerged from the "two-hit" hypothesis of Knudson and the cell hybrid studies of Harris et al. (Proc. Natl. Acad. Sci. USA 68, 820-823 (1971)); Nature 223, 363-368 (1969)). The nucleotide sequence of the p53 gene is depicted in Table 3.

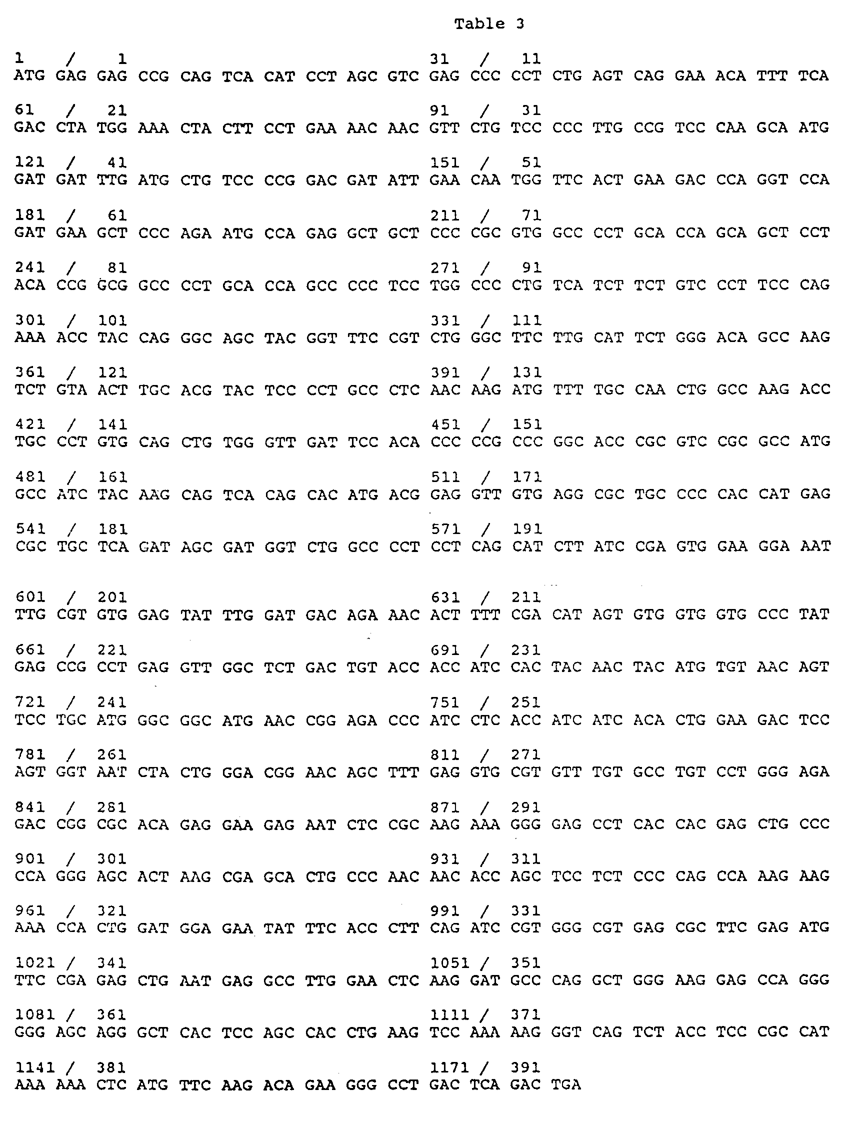

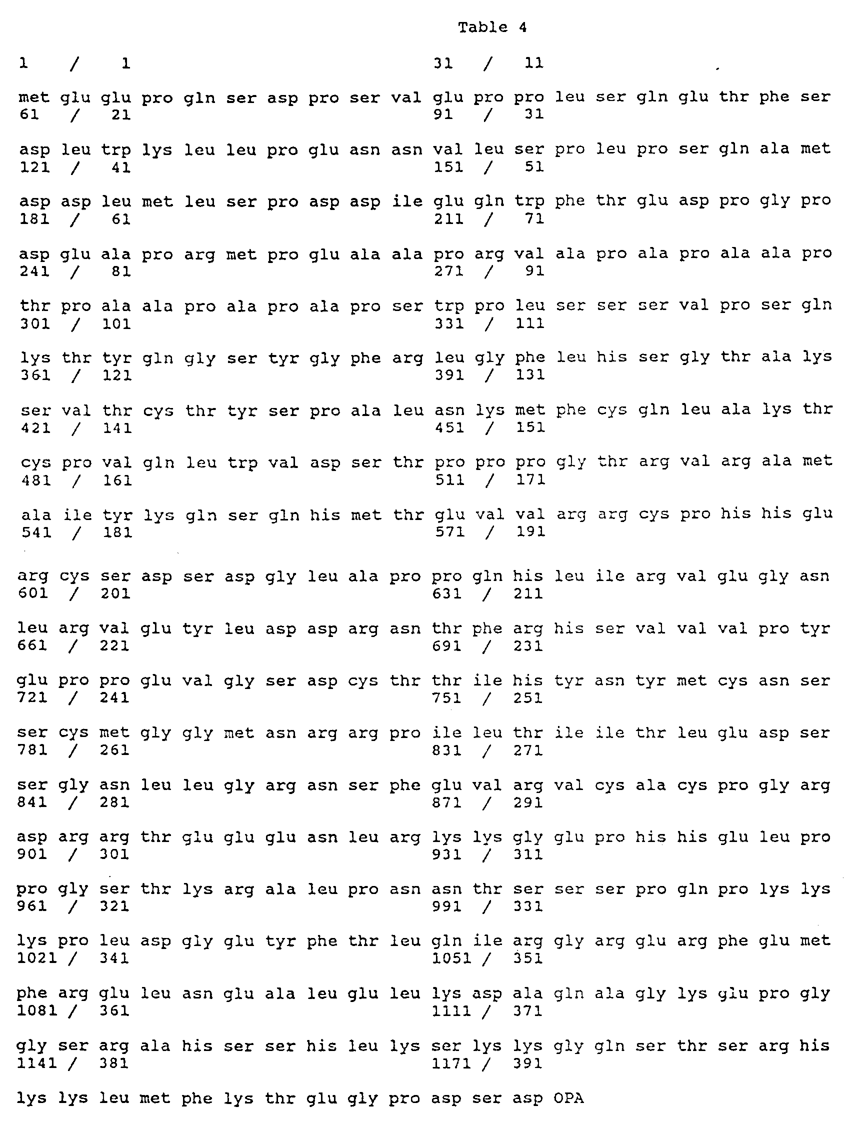

p53 (Table 4) was originally identified as a mammalian cellular protein that binds to SV40T antigen, a property that is also shared by RB protein. Deletions or rearrangements of the murine or human gene encoding p53 were found in Friend virus-induced murine erythroleukemias, and in human osteosarcomas, lung carcinomas, lymphomas and leukemias. On the other hand, many human breast, lung and colon carcinomas expressed high levels of aberrant p53 species with markedly prolonged half-lives due to certain point mutations in the p53 gene (Genes Devel. 4, 1-8 (1990)). These observations suggest that mutation of p53 contributes to human oncogenesis. p53 was originally considered to be an oncogene because it was known that it could transform primary rat embryo fibroblasts in concert with an activated ras gene. However, the observation of p53 deletions, and point mutations scattered over several exons, also suggested that p53 might be a tumor suppressor gene, i.e., a gene that was inactivated by mutation. Indeed, Finlay et al. and Eliyahu et al. (Cell 57, 1083-1093 (1989)) Proc. Natl. Acad. Sci U.S.A. 86, 8763-8767 (1989)) found that cotransfection of murine wild-type p53 DNA could reduce the transformation efficiency of transfected oncogenes in rat embryo fibroblasts, whereas mutated p53 DNA enhanced such transformation. The dominant transforming effect was presumed to be due to a "dominant negative" activity of mutated p53 protein that somehow blocked the growth-restricting function of wild-type p53 protein in cells. This model suggested that the relative quantity of mutated to wild-type p53 could determine the transformed phenotype, but gene dosage could not be tightly controlled in these transfection studies.

Because of such technical questions, as well as the possibility of species-specific differences in p53 function and the uncertain relevance of transformed animal cells to human neoplasia, it was determined that the biological properties of p53 in the human system should be reassessed. It was recognized that an ideal host cell for these studies would allow the experimental manipulation of single copies of mutated or wild-type p53 alleles. However, most cultured human cells contain endogenous and possibly mutated p53 alleles that are not accessible to external control. The human osteosarcoma cell line Saos-2 was therefore chosen because it has no endogenous p53 due to complete deletion of its gene. Recombinant retroviruses derived from Moloney murine leukemia virus (Mo-MuLV) were used to introduce mutated and/or wild-type p53 under LTR promoter control. Cell clones isolated after infection and selection carried only a single integrated provirus of each type, and multiple clones were analyzed to exclude positional effects. A comprehensive assessment of biological properties of these clones included morphology, growth rates and saturation density in culture, colony formation in soft agar, and tumorigenicity in nude mice.

### Preparation of mutated and wild-type p53 recombinant retroviruses.

As a reference standard for human wild-type p53, the genomic DNA sequence of Lamb and Crawford (Mol. Cell. Biol. 6, 1379-1385 (1986)) was used. Potentially wild-type p53 cDNA was isolated from fetal brain RNA by the method of RT-PCR, and was cloned into plasmid. In cloning of wild type p53 (p53B) cDNA about 5µg of fetal brain RNA were mixed with 1.5µg of oligo(dT) primer and 60 units of avian myeloblastosis virus reverse transcriptase in cDNA buffer (50 mM Tris-HCl, pH 8.0, 80mM KCl, 5mM MgCl₂, 1mM each dATP, dGTP, dTTP, and dCTP). The reaction mixture was incubated for 90 min at 42°C. After reaction, RNA was degraded with 0.5N NaOH, and single-stranded cDNA was precipitated with ethanol. PCR amplification was carried out with one-tenth of the cDNA, 100 ng of each oligonucleotide primer (5'-TGCAAGCTTTCCACGACGGTGACACGCT-3' and 5-AGTGCAGGCCAACTTGTTCAGTGGA-3'), and 5 U of Taq polymerase in PCR buffer (50 mM KCl, 10 mM Tris-HCl, pH 8.3, 1.5 mM MgCl₂, and 0.001% gelatin) for 40 cycles in a programmable heat block (Ericomp, San Diego, CA). Each cycle included denaturation at 93°C for 1 minute, reannealing at 62° for 80 seconds, and primer extension at 72°C for 3 minutes. PCR products were extracted with phenol and precipitated with ethanol. The precipitate was dissolved in H₂O and digested with restriction enzymes (Hind III and Sma 1). The p53 cDNA fragment was subcloned into virus vector to form Vp53B-Neo. Subcloned p53B was sequenced by using the dideoxy chain termination method (Proc. Natl. Acad. Sci. U.S.A. 74, 5463 (1977)).

The insert in one clone (designated p53B) was entirely sequenced (-1300 bp) to reveal a wild-type deduced amino acid sequence despite two silent nucleotide replacements (FIG. 1A). Another p53 cDNA clone (p53E), isolated from an epidermoid carcinoma cell line A431, was also sequenced, and was found to contain a point mutation at codon 273 that replaced Arg with His (FIG. 1A). This is a functionally significant mutation that has also been identified in p53 from two other tumor cell lines. In addition, a neutral sequence polymorphism in codon 72 (FIG. 1A) encoded either an Arg (p53B) or a Pro (p53E). This common amino acid polymorphism, which is without known functional significance, resulted in faster migration of p53B than p53E protein by SDS-PAGE, and was therefore exploited to distinguish between these proteins when they were coexpressed in the same cell.

p53E and p53B were then inserted into a Mo-MuLV-based retroviral vector containing neo as a selectable marker gene to form Vp53E-Neo and Vp53B-Neo viral genomes, respectively (FIG. 1B). In addition, to facilitate double replacement, Vp53B-Hygro was made by inserting p53B into a similar vector containing the gene which is known to confer resistance to hygromycin. Stocks of Vp53E-Neo, Vp53B-Neo and Vp53B-Hygro viruses were produced, utilizing conventional methods, with titers of about 1 x 10⁵, 2 x 10⁴, and 1 x 10⁵, respectively. Expression of p53 proteins from the viruses was initially assessed in the murine NIH3T3-derived packaging line, PA12, that was used for virus production. Mutated and wild-type human p53 proteins were detected in their respective virus-producing cells, with the expected difference in migration by SDS-PAGE (FIG. 2A). Because spontaneous mutation of p53 may occur frequently in cultured cells, two additional biochemical properties of these p53 proteins were examined. These were their cellular half-lives, and their ability to bind to T antigen. p53B protein had a half-life of 20-30 minutes compared to 4 to 5 hours for p53E protein (FIG. 2B), consistent with published reports on the half-lives of wild-type and mutated p53 proteins. When virus-producing cells were transfected with a plasmid expressing large amounts of SV40T antigen, and lysates were immunoprecipitated with anti-p53 or anti-T antibodies, T antigen was coprecipitated with p53B but not p53E protein, indicating that only p53B protein could bind to T. These results together suggested that p53B-containing viruses expressed wild-type, and that p53E containing virus expressed mutated p53.

### Expression of exogeneous p53 in osteosarcoma cells.

Osteosarcoma cell line Saos-2, which contains no endogenous p53, because of deletion of its gene, provides a clean background for functional studies of p53. In previous experiments, Saos-2 cells infected with parental viruses containing only neomycin- or hygromycin-resistance genes showed no changes in morphology and growth rate compared to uninfected cells, suggesting that drug selection did not have a significant influence on their neoplastic properties. Saos-2 cells infected with comparable titers of either Vp53E-Neo, Vp53B-Neo, or Vp53B-Hygro in the presence of the appropriate selective agent each yielded similar numbers of drug-resistant colonies. Most colonies could be individually propagated into mass cultures, with the notable observation that Vp53B-infected cells grew much more slowly than Vp53E-infected cells. Vp53E-infected clones uniformly expressed high levels of p53E protein (FIG. 3A). Of 30 Vp53B-infected clones examined, about 80% expressed detectable p53B protein (FIG. 3B). Two each of Vp53E-Neo and Vp53B-Hygro clones were randomly selected for a second infection by the other virus, and double-infected clones were isolated and propagated as above. These clones coexpressed both p53E and p53B protein (FIG. 3C). To again verify that p53B protein in these cells was not secondarily mutated, p53B-expressing clones were transfected with the SV40 antigen plasmid, and lysates immunoprecipitated as described above (FIG. 4). Anti-p53 antibody coprecipitated T in each clone, but anti-T antibody coprecipitated only p53B, even in cells expressing both p53B and p53E. The half-life of p53B in Saos-2 was also measured, and was similar to that of p53B in PA 12 cells. These data again support the notion that Vp53B-infected Saos-2 clones expressed wild-type p53.

### Mutated p53 conferred a limited growth advantage to Saos-2 cells in culture.

Five randomly chosen clones that stably expressed p53E protein (p53EN-1 to 5) were compared to parental cells in terms of morphology (FIG. 5), growth rate (as doubling time, FIG. 6A), saturation density (FIG. 6B), soft-agar colony formation and tumorigenicity in nude mice were determined. In Tables 1 and 2, the results of soft-agar colony formation and tumorigenicity in nude mice, respectively are tabulated.

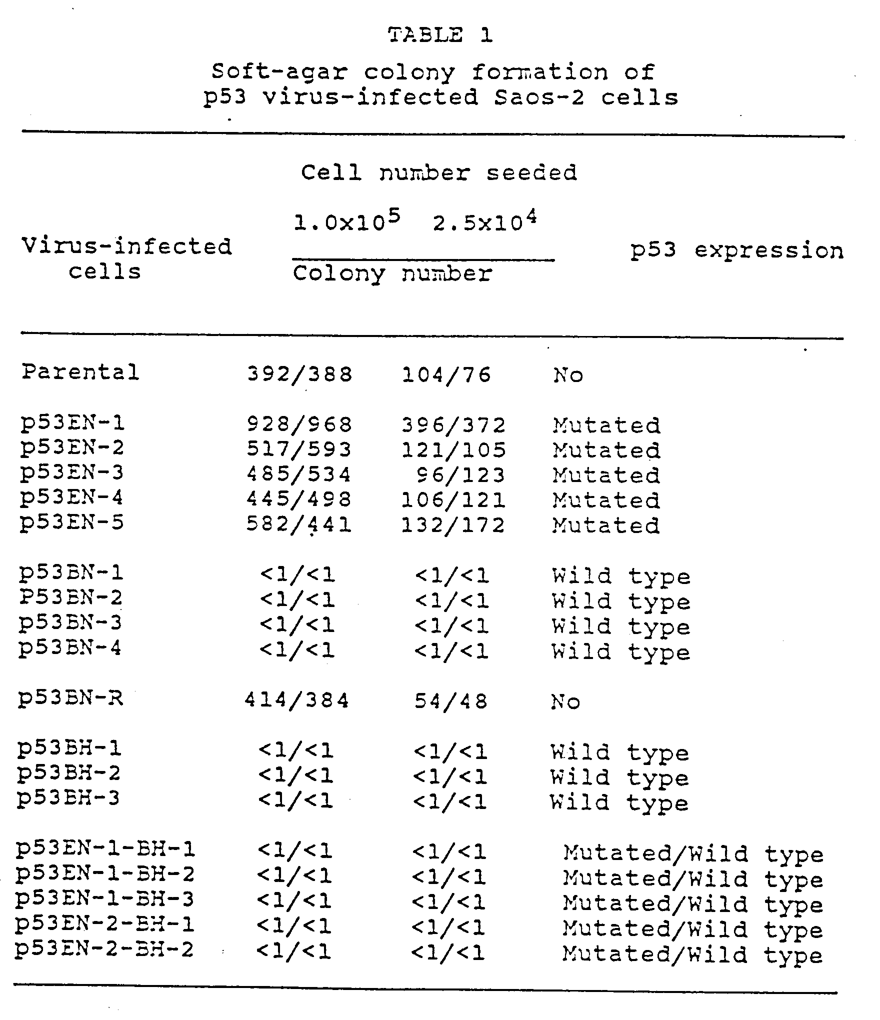

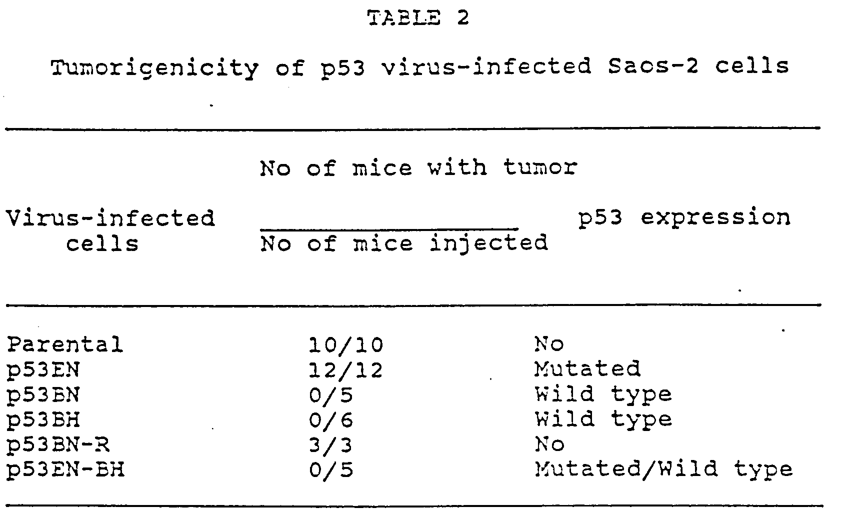

A difference in morphology was observed only under conditions of cell crowding, where cells of EN clones were far smaller and more refractile than parental cells (FIG. 5B). Correlatively, saturation density of the former was 4- to 5-fold greater than that of parental cells (FIG. 6B). This relative growth advantage was seen despite similar doubling times as measured under sparse growth conditions (FIG. 6A). Four EN clones and parental cells shared similar efficiencies in soft-agar colony formation (Table 1) and tumorigenicity in nude mice (Table 2). One clone, p53EN-1, had noticibly augmented abilities in both respects; in particular, it reliably formed large tumors from as few as 5 x 10⁵ injected cells. This discrepancy was considered to be within the range of clonal variability expected among tumor cells. In summary, these results suggested that mutated p53 functioned in the absence of wild-type p53 to confer a limited growth advantage (higher saturation density) to Saos-2 cells in culture. In many other aspects of the neoplastic phenotype, the presence of point-mutated p53 was essentially equivalent to complete absence of p53.

### Wild-type p53 suppressed the neoplastic properties of Saos-2 cells.

In comparison to parental Saos-2 cells, clones expressing p53B were invariably enlarged and flattened (FIG. 5), and has prolonged doubling times in culture of about 70 hours rather than 30-36 hours for parental or EN cells (FIG. 6A). Remarkably, the efficiency of soft-agar colony formation was reduced to less than the threshold for detecting a single colony, whereas parental cells and EN cells formed hundreds of colonies under the same conditions (Table 1). Injection of 1 x 10⁷ cells of each of seven p53B-expressing clones into the flanks of nude mice resulted in the formation of no tumors after 8-10 weeks, even while the same number of parental or p53E-expressing cells formed tumors in all contralateral flanks (Table 2). These findings could not be explained by a peculiar effect of viral infection and selection because one clone, Vp53BN-R, derived from Vp53B-Neo-infected cells but lacking detectable expression of p53B, had a phenotype indistinguishable from parental cells (Tables 1 and 2). The -50% reduction of growth rate of cultured Saos-2 cells by p53B was insufficient to account for the complete loss of tumorigenicity and soft-agar colony formation, implying that wild-type p53 specifically suppressed the neoplastic phenotype of these cells. These results suggested that loss of wild-type p53 was a significant event during the genesis of this tumor line, and, by extension, of other osteosarcomas, with mutated endogenous p53 genes.

### Wild-type p53 was dominant to mutated p53 in a two-allele configuration.

Because both mutated and wild-type p53 proteins were apparently functional in Saos-2 cells, it was of interest to determine whether both activities could be simultaneously coexpressed, whether they cancelled out one another, or whether one activity was clearly dominant over the other. The configuration of one wild-type and one mutated allele was most relevant to natural human tumorigenesis, because this is a necessary intermediate step on the pathway toward complete loss of wild-type p53. Infection of two different p53E-expressing clones with Vp53B-Hygro yielded 22 hygromycin-resistant clones, of which 15 coexpressed both p53B and p53E. To determine the number of integrated copies of each virus present in these clones, genomic DNA of three clones derived from p53EN-1 cells infected with Vp53B-Hygro was analyzed by Southern blotting (FIG. 7). Hybridization with neo as a probe showed a single, common junctional fragment in all three clones, indicating the presence of a single integrated copy of Vp53E-Neo in p53EN-1 cells (FIG. 7A). Hybridization with hygro showed a single, unique junctional fragment in each clone, indicating the presence of single, independently integrated copies of Vp53B-Hygro in p53EN-BH clones (FIG. 7B). Single integrations were expected, based on previous use of a related recombinant retrovirus all comparable titers. These findings confirmed that p53EN-BH clones indeed contained one integrated copy of each virus, and that both exogenous p53 genes were expressed (FIG. 3). By criteria of morphology, growth rate, saturation density, soft-agar colony formation, and tumorigenicity in nude mice, double-replacement clones were indistinguishable from clones expressing only p53B (FIGS. 5 and 6, Tables 1 and 2). Cells obtained by infecting in the other order, i.e., p53B-expressing cells infected with Vp53E-Neo, had the same phenotype. Complete dominance of wild-type p53 activity was observed despite the -10-fold lower quantities of wild-type than mutated p53 in these cells (FIG. 3). These results indicate that p53 can contribute to tumorigenesis only after loss of both wild-type alleles. They also indicate that restoration of wild-type p53 in tumor cells may have a suppressive effect, even in the presence of mutated p53 alleles.

### Function of p53 as a tumor suppressor.

Introduction of wild-type p53 in human osteosarcoma cells lacking p53 expression clearly suppressed their neoplastic phenotype, indicating that p53 can function as a tumor suppressor gene in this system. Conversely, insertion of mutated p53 into these cells augmented one aspect of their growth in culture (saturation density), thereby showing that mutated p53 retains a limited function, albeit one that was overridden by wild-type p53. These results are broadly consistent with those of other investigators who have addressed the influence of wild-type murine p53 on oncogene-mediated transformation of primary rat embryo fibroblasts. In these studies, cotransfection of plasmid DNA containing the wild-type p53 gene markedly reduced the transformation efficiency of several activated oncogenes, either singly or in combinations such as ras + myc or ras + E1A. Mutated p53 did not have this suppressive effect, and instead modestly boosted transformation efficiency. Wild-type p53 was also effective in reducing transformation by mutated p53 in concert with other oncogenes, suggesting "dominance" of the wild-type suppression function. Colonies recovered after transfection with wild-type p53 DNA either failed to express exogenous p53, or expressed only mutated p53.

Thus, it appeared that expression of exogenous, wild-type p53 was incompatible with formation of transformed colonies. These data suggested that wild-type murine p53 could function as a suppressor of transformed cells, although a nonspecific, toxic effect of wild-type p53 was not easily excluded. In contrast, in development of the present invention, transformed cells were utilized, and growing clones with altered phenotype that stably expressed exogenous, wild-type p53 were obtained. These date in human cells, and the previous studies in mouse, together indicate that the tumor suppression function of p53 is a specific and fundamental property conserved across species boundaries.

### The nature of p53 mutation.

It is known that murine p53 genes cloned from many cultured cell lines have point mutations that cluster in five conserved regions. This class of mutation was responsible for the initial impression that p53 was a dominant oncogene, because such p53 DNA fragments or constructs were active in promoting transformation of rodent cells in a variety of assays. Furthermore, protein products of mutated p53 genes have common antigenic and biochemical characteristics that differ from wild-type p53 protein, including a prolonged half-life that results in abnormally high cellular p53 protein levels. These features are quite reminiscent of other dominant oncogenes like myc and ras. On the other hand, gross deletions or rearrangements of the p53 gene, incompatible with expression of a gene product have been found in Friend-virus induced murine erythroleukemias, (Nature 314, 633-636 (1985)). Such mutations are considered to be characteristic of so called tumor suppressor genes, and serve to inactivate their normal function. To explain how both kinds of mutation could impart the same oncogenic phenotype, it was proposed that wild-type p53 indeed functioned to suppress tumor formation, and that the many known point mutations of p53 actually served to inactivate this function. To explain the dominant transforming activity of mutated p53 genes in primary cells, it was necessary to hypothesize that mutated p53 protein somehow inactivated endogenous, wild-type p53 protein. This "dominant negative" effect might occur by inhibitory interactions between mutated and wild-type proteins, (Nature, 329, 219-222 (1987)). Further interpretation of these studies was limited by the technical drawbacks of transfection, and by the uncertain role of endogenous p53 in primary cells.

In the human system, Vogelstein and colleagues have shown in elegant studies that deletions and point mutations of p53 can coexist in colorectal, lung or breast carcinomas. Loss of heterozygosity of polymorphic markers in chromosome region 17p is seen frequently in these tumors, corresponding to the loss of one copy of the p53 gene (by deletion or mitotic nondisjunction). The remaining p53 allele is often affected by somatic point mutations in conserved regions. The end result is the loss of both wild-type p53 alleles from tumor cells. The same loss also occurs in human osteosarcomas and hepatocellular carcinomas by deletion of both p53 alleles. Complete loss of wild-type alleles is highly analogous to findings with the retinoblastoma gene, and support the idea that p53 is a tumor suppressor gene. However, Nigro et al., J. M. Nigro et al., Nature 342, 705-708 (1989), described one colorectal carcinoma coexpressing both mutated (Asp²⁸¹ --> Gly) and wild-type p53 alleles. The existence of this tumor was interpreted as favoring an oncogenic activity of a single mutated p53 allele in the presence of wild-type p53; loss of the second, wild-type allele would contribute to progression of the tumor.

In the present invention, it has been found that the phenotype of Saos-2 cells with single copies of wild-type and mutated p53 alleles was indistinguishable from cells expressing wild-type p53 alone, suggesting that wild-type p53 is dominant to mutated p53 in two-allele configuration. Given this result, other explanations for the discrepant colon carcinoma case may be considered: 1) an intermediate stage of p53 mutation was coincidentally captured, and p53 had not yet contributed to the neoplastic properties of this tumor; and 2) the "wild-type" p53 allele in this tumor actually carried a functionally important mutation outside of the region sequenced (exons 5-9). On the other hand, it is possible that certain mutated p53 alleles behave differently than others, or that mutated p53 alleles function differently in other types of tumor cells than in our model Saos-2 system. These possibilities can be addressed by replicating experiments with other mutated p53 genes and other p53-negative cells.

Confusion in previous studies about the interaction between wild-type and mutated p53 have clouded an essential question: is mutated p53 completely functionless, i.e., is it equivalent to its complete deletion, or does it retains some limited function? It is concluded that the latter case is more probable. A single copy of mutated p53 increased in saturation density of Saos-2 cells, and of course this effect could not be mediated by inactivation of endogenous p53. Similarly, Wolf et al., (Cell 38, 119-126 (1984)), introduced what was probably a mutated p53 gene into AB-MuLV-transformed murine cells that lacked endogenous p53 expression, and found that their oncogenic potential was increased. Therefore, mutated p53 alleles may confer a growth advantage or a more malignant phenotype in vivo to tumor cells without wild-type p53.

The findings that mutated p53 has a biological function, and that its function is recessive to that of wild-type p53, are inconsistent with the hypothesis of a dominant negative effect, at least as it applies in natural human tumorigenesis. The dominant transforming properties of mutated murine p53 alleles may be due to the high copy numbers of genes introduced by transfection, and the resulting massive overexpression of mutated p53. Under these circumstances, even its limited intrinsic activity may be sufficient to contribute to a transformed phenotype.

### Mechanisms of p53 function.

The physiological or biochemical functions of p53 are now known with certainty. In nontransformed cells, p53 synthesis and mRNA transcription increase dramatically during the transition from G₀/G₁ to S phase, indirectly suggesting a role in cell cycle regulation. Recent evidence also points to a possibly related activity in regulating DNA replication. Studies on suppression of the neoplastic phenotype may provide general parameters for understanding the normal function of p53. It is clear, for example, that p53 is not required for progression of the cell cycle, nor is its presence necessarily preventive of cell growth and division. Therefore, it may participate in regulation of these basic cellular processes in response to external growth or differentiation signals. Wild-type and mutated p53 can differ by a single amino acid yet have opposing functions in the cell. Under conditions of equal gene dosage, wild-type p53 is able to override the influence of mutated p53 despite a 10-fold molar excess of the latter. These observations may be explained by competition of wild-type and mutated p53 for common cellular targets, for which wild-type p53 is much more avid. In this model, wild-type and mutated p53 would transmit opposite growth signals to these targets, with total absence of p53 perhaps an intermediate signal. Alternatively, mutated p53 may act in an independent pathway to promote selective features of the neoplastic phenotype.

### Genetic mechanisms of p53 inactivation.

The dominance of wild-type over mutated p53 in a two-allele configuration implies that both wild-type p53 alleles must be lost for an oncogenic effect. In this respect, p53 conforms to a model of tumor suppressor gene inactivation that can be understood in the case of the retinoblastoma gene. Complete loss of the RB gene product is so far universal in retinoblastomas, and wild-type and mutated RB alleles have not been observed to coexist in tumor cells. These findings suggest that RB contributes to oncogenesis only after its complete inactivation. On the other hand, many tumor cells have normal RB expression, and are neoplastic presumably because of mutations in other genes. In such RB+ tumor cells, introduction of additional, exogenous RB may have little or no effect; for example, it has been found that RB+ U20S osteosarcoma cell lines with wild-type p53 alleles are not known to exist. The results obtained to date indicate that p53 has broad suppression activity in several types of human tumors. Thus, the suppression effect of exogenous RB or p53 may occur only in tumor cells with inactivated RB or p53 genes. These shared properties of RB and p53 reinforce the tumor suppressor gene concept, including the possible clinical utility of their replacement in appropriate tumor cells.

### Summary.

Mutations of the gene encoding p53, a 53 kD cellular protein, are found frequently in human tumor cells, suggesting a crucial role for this gene in human oncogenesis. In order to model the stepwise mutation or loss of both p53 alleles during human oncogenesis, a human osteosarcoma cell line, Saos-2 was utilized that lacked endogenous p53 due to complete deletion of the gene. Single copies of exogenous p53 genes were then introduced by infecting cells with recombinant retroviruses containing either wild-type or point-mutated versions of the p53 cDNA sequence. It was found that 1) expression of wild-type p53 suppresses the neoplastic phenotype of Saos-2 cells; 2) expression of mutated p53 confers a limited growth advantage to cells in the absence of wild-type p53; and 3) wild-type p53 is phenotypically dominant to mutated p53 in a two-allele configuration. These results indicate that, as with the retinoblastoma gene, mutation of both alleles of the p53 gene is essential for its role in oncogenesis.

While particular embodiments of the present invention have been disclosed, it is to be understood that various different modifications are possible and are contemplated within the true spirit and scope of the appended claims. There is no intention, therefore, of limitations to the exact abstract or disclosure herein presented.

## Claims (Claims for the following Contracting State(s): AT, BE, CH, DE, DK, FR, GB, IT, LI, LU, NL, SE)

1. A medicament for use in the suppression of the neoplastic phenotype of a cancer cell having no endogenous wild-type p53 protein comprising the wild-type p53 gene comprising the sequence of Table 3.

2. A medicament of claim 1 wherein the wild-type p53 gene is included in a retroviral vector.

3. A medicament of claim 1 wherein the cancer cell having no endogenous wild-type p53 protein lacks the wild-type p53 tumor suppressor gene.

4. A medicament of claim 1 wherein the cancer cell having no endogenous wild-type p53 protein has a mutated p53 tumor suppressor gene.

5. A medicament of claim 1 wherein the cancer cell having no endogenous wild-type p53 protein is an osteosarcoma cell, lung carcinoma cell, lymphoma cell, leukemia cell, soft tissue sarcoma cell, breast carcinoma cell, bladder carcinoma cell or prostate carcinoma cell.

## Claims (Claims for the following Contracting State(s): ES)

1. A process for preparing a medicament for use in the suppression of the neoplastic phenotype of a cancer cell having no endogenous wild-type p53 protein, said process comprising mixing the wild-type p53 gene comprising the sequence of Table 3, with a pharmaceutically acceptable carrier.

2. A process of claim 1 wherein the wild-type p53 gene is included in a retroviral vector.

3. A process of claim 1 wherein the cancer cell having no endogenous wild-type p53 protein lacks the wild-type p53 tumor suppressor gene.

4. A process of claim 1 wherein the cancer cell having no endogenous wild-type p53 protein has a mutated p53 tumor suppressor gene.

5. A process of claim 1 wherein the cancer cell having no endogenous wild-type p53 protein is an osteosarcoma cell, lung carcinoma cell, lymphoma cell, leukemia cell, soft tissue sarcoma cell, breast carcinoma cell, bladder carcinoma cell or prostate carcinoma cell.

## Patentansprüche (Patentansprüche für folgende(n) Vertragsstaat(en): AT, BE, CH, DE, DK, FR, GB, IT, LI, LU, NL, SE)

1. Arzneimittel zur Verwendung bei der Suppression des neoplastischen Phänotyps einer Krebszelle, die kein endogenes Wildtyp-p53-Protein aufweist, wobei dieses Arzneimittel das Wildtyp-p53-Gen enthält, das die Sequenz aus Tabelle 3 umfaßt.

2. Arzneimittel nach Anspruch 1, wobei das Wildtyp-p53-Gen in einem retroviralen Vektor enthalten ist.

3. Arzneimittel nach Anspruch 1, wobei der Krebszelle, die kein endogenes Wildtyp-p53-Protein aufweist, das Wildtyp-p53-Tumorsuppressorgen fehlt.

4. Arzneimittel nach Anspruch 1, wobei die Krebszelle, die kein endogenes Wildtyp-p53-Protein aufweist, ein mutiertes p53-Tumorsuppressorgen aufweist.

5. Arzneimittel nach Anspruch 1, wobei die Krebszelle, die kein endogenes Wildtyp-p53-Protein aufweist, eine Osteosarkomzelle, Lungenkarzinomzelle, Lymphadenomzelle, Leukämiezelle, Weichteilgewebesarkomzelle, Brustkrebszelle, Blasenkrebszelle oder Prostatakarzinomzelle ist.

## Patentansprüche (Patentansprüche für folgende(n) Vertragsstaat(en): ES)

1. Verfahren zur Herstellung eines Arzneimittels zur Verwendung bei der Suppression des neoplastischen Phänotyps einer Krebszelle, die kein endogenes Wildtyp-p53-Protein aufweist, wobei dieses Verfahren das Mischen des Wildtyp-p53-Gens, das die Sequenz aus Tabelle 3 umfaßt, mit einem pharmazeutisch annehmbaren Träger beinhaltet.

2. Verfahren nach Anspruch 1, wobei das Wildtyp-p53-Gen in einem retroviralen Vektor enthalten ist.

3. Verfahren nach Anspruch 1, wobei der Krebszelle, die kein endogenes Wildtyp-p53-Protein aufweist, das Wildtyp-p53-Tumorsuppressorgen fehlt.

4. Verfahren nach Anspruch 1, wobei die Krebszelle, die kein endogenes wildtyp-p53-Protein aufweist, ein mutiertes p53-Tumorsuppressorgen aufweist.

5. Verfahren nach Anspruch 1, wobei die Krebszelle, die kein endogenes Wildtyp-p53-Protein aufweist, eine Osteosarkomzelle, Lungenkarzinomzelle, Lymphadenomzelle, Leukämiezelle, Weichteilgewebesarkomzelle, Brustkrebszelle, Blasenkrebszelle oder Prostatakarzinomzelle ist.

## Revendications (Revendications pour l'(les) Etat(s) contractant(s) suivant(s): AT, BE, CH, DE, DK FR, GB, IT, LI, LU, NL, SE)

1. Médicament à utiliser dans la suppression du phénotype néoplasique d'une cellule cancéreuse n'ayant pas de protéine p53 de type sauvage endogène comprenant le gène p53 de type sauvage comprenant la séquence du tableau 3.

2. Médicament selon la revendication 1, dans lequel le gène p53 de type sauvage est inclus dans un vecteur rétroviral.

3. Médicament selon la revendication 1, dans lequel la cellule cancéreuse n'ayant pas de protéine p53 de type sauvage endogène est dépourvue de gène suppressif de tumeurs p53 de type sauvage.

4. Médicament selon la revendication 1, dans lequel la cellule cancéreuse n'ayant pas de protéine p53 de type sauvage endogène a un gène suppressif de tumeurs p53 muté.

5. Médicament selon la revendication 1, dans lequel la cellule cancéreuse n'ayant pas de protéine p53 de type sauvage endogène est une cellule d'ostéosarcome, une cellule de cancer du poumon, une cellule de lymphome, une cellule leucémique, une cellule de sarcome des tissus mous, une cellule de cancer du sein une cellule de cancer de la vessie ou une cellule de cancer de la prostate.

## Revendications (Revendications pour l'(les) Etat(s) contractant(s) suivant(s): ES)

1. Procédé de préparation d'un médicament à utiliser dans la suppression du phénotype néoplasique d'une cellule cancéreuse n'ayant pas de protéine p53 de type sauvage endogène, ledit procédé comprenant le mélange du gène p53 de type sauvage comprenant la séquence du tableau 3 avec un véhicule pharmaceutiquement acceptable.

2. Procédé selon la revendication 1, dans lequel le gène p53 du type sauvage est inclus dans un vecteur rétroviral.

3. Procédé selon la revendication 1, dans lequel la cellule cancéreuse n'ayant pas de protéine p53 de type sauvage endogène est dépourvue de gène suppressif de tumeurs p53 de type sauvage.

4. Procédé selon la revendication 1, dans lequel la cellule cancéreuse n'ayant pas de protéine p53 de type sauvage endogène a un gène suppressif de tumeurs p53 muté.

5. Procédé selon la revendication 1, dans lequel la cellule cancéreuse n'ayant pas de protéine p53 de type sauvage endogène est une cellule d'ostéosarcome, une cellule de cancer du poumon, une cellule de lymphome, une cellule leucémique, une cellule de sarcome des tissus mous, une cellule de cancer du sein, une cellule de cancer de la vessie ou une cellule de cancer de la prostate.
